Europäisches Patentamt

European Patent Office

Office européen des brevets

(19)

(11) **EP 1 457 256 A1**

(12) **EUROPEAN PATENT APPLICATION**
published in accordance with Art. 158(3) EPC

| | |
|---|---|
| (43) Date of publication:<br>**15.09.2004 Bulletin 2004/38** | (51) Int Cl.[7]: **B01J 20/24**, A61K 31/731,<br>A61K 31/732, A61K 31/734,<br>A61P 3/00 |
| (21) Application number: **02805475.7** | |
| (22) Date of filing: **18.12.2002** | (86) International application number:<br>**PCT/JP2002/013248** |
| | (87) International publication number:<br>**WO 2003/053565 (03.07.2003 Gazette 2003/27)** |

| | |
|---|---|
| (84) Designated Contracting States:<br>**AT BE BG CH CY CZ DE DK EE ES FI FR GB GR<br>IE IT LI LU MC NL PT SE SI SK TR**<br>Designated Extension States:<br>**AL LT LV MK RO** | • **IENAKA, T., c/o Muromachi Chemical Co., Ltd<br>Omuta-shi, Fukuoka 836-0895 (JP)**<br>• **NODA, H.,<br>c/o Sanwa Kagaku Kenkyusho Co., Ltd<br>Nagoya-shi, Aichi 461-8631 (JP)**<br>• **TOMOTO, A.,<br>c/o Sanwa Kagaku Kenkyusho Co., Ltd<br>Nagoya-shi, Aichi 461-8631 (JP)** |
| (30) Priority: **21.12.2001 JP 2001388788** | |
| (71) Applicant: **Muromachi Chemical Co., Ltd<br>Omuta-shi, Fukuoka 836-0895 (JP)** | (74) Representative: **W.P. THOMPSON & CO.<br>Eastcheap House<br>Central Approach<br>Letchworth Garden City,<br>Hertfordshire SG6 3DS (GB)** |
| (72) Inventors:<br>• **SANO, H., c/o Muromachi Chemical Co., Ltd<br>Omuta-shi, Fukuoka 836-0895 (JP)** | |

(54) **ADSORBENT FOR PHOSPHORIC ACID**

(57) Provided is an adsorbent for phosphoric acid which can be produced with ease, exhibits high adsorbability of phosphoric acid and is free from any problems in use thereof. The adsorbent for phosphoric acid which includes a water-insoluble reaction product formed by mixing an iron ion and a natural polysaccharide having a carboxylic functional group or a sulfuric acid functional group such as alginate, pectin and carrageenan in a solution, or a dried product thereof. The additional incorporation of a hydrophilic polymer such as agar into the adsorbent prevents the reduction in adsorbability of phosphoric acid in the case where a trivalent iron ion is used to produce the dried product of a water-insoluble reaction product.

FIG. 1

EP 1 457 256 A1

## EP 1 457 256 A1

**Description**

TECHNICAL FIELD TO WHICH THE INVENTION BELONGS

The present invention relates to an adsorbent for phosphoric acid which includes a water-insoluble reaction product formed by mixing a divalent or trivalent iron ion and a natural polysaccharide having a carboxylic acid functional group or a sulfuric acid functional group and the invention relates, for example, to fields of water treatment including supply water and sewage as well as drinking water, fields of pretreatments of foods, chemicals, and analytical samples, or fields of pharmaceuticals.

PRIOR ART

Phosphoric acid is an essential biomolecule which is known to widely occur in natural and artificial environments and plays an important role in biological activities of living organisms. However, in recent years, discharge of excessive amounts of phosphoric acid in the environment, balance disorders in uptake of phosphoric acid and the like have been pointed out. To solve the problems, various adsorbents and coagulants which have an ability to adsorb phosphoric acid have been used.

In particular, materials and techniques which utilize well-known performance capabilities of phosphoric acid to be able to specifically bind with transition metals such as iron and aluminum have been widely utilized. For example, 1) those which include an ion exchange resin, activated carbon and a porous substance such as porous chitosan having carried thereon transition metals such as iron and aluminum, and 2) coagulative precipitants using aluminum and a high polymer material are known (WADA, Hiromutsu, "Technology of Water Making", Chijinshokan; JP 2002-282686 A; JP 6-157324 A; JP 5-155776 A).

However, the existing methods and materials have the following drawbacks.

(1) They have drawbacks in properties that removal of phosphoric acid from high concentration organic solutions is difficult.

(2) Additional carrying of a heavy metal compound on a conventional porous material or cellulose upon fabrication incurs a great time cost and load on the environment. For example, production of ion exchange resins uses noxious organic solvents. Activated carbon requires a large amount of caloric values upon production and generates heat and carbon dioxide.

(3) Upon use and treatment of wastes, they require a facility such as a sedimentation tank or a filtration tank. Those which use aluminum have the possibility of causing Alzheimer's disease. In addition, those based on materials obtained by use of organic synthesis, as observed in the case of, for example, ion exchange resins which include a polystyrene reaction product as a base material, have the danger of generating environmental hormones or other noxious substances when a portion of the polymerized polymer is released.

Diet therapy and an oral adsorbent for phosphorus are used for the therapy of hyperphosphatemia. In principal, administration of vitamin $D_3$ preparations, which is a phosphorus absorbing substance, is stopped, low phosphorus diet is administered, and a sufficient amount of dialysis is carried out. In addition, as a general rule, the oral adsorbent for phosphorus is administered when a control serum phosphorus level is insufficient.

Oral adsorbents for phosphorus include aluminum preparations (aluminum hydroxide), calcium preparations (calcium carbonate, calcium acetate) and magnesium preparations (magnesium carbonate) for the purpose of intestinal absorption of phosphoric acid. The aluminum preparations have problems of side effects caused by absorption and accumulation of aluminum, i.e., osteomalacia and aluminum encephalosis. The calcium preparations have lower adsorption capabilities than the aluminum preparations and must be taken in a large amount, so that they have problems of side effects of hypercalcemia. Also, the magnesium preparations, like the calcium preparations, have problems of side effects of hypermagnesemia.

Examples of use of iron for the purpose of therapy of hyperphosphatemia utilizing the action of iron to adsorb phosphoric acid include a complex of chitosan with added iron ion as described in JP 6-157324 A, an acetylated iron chitosan complex as described in JP 7-2903 A, and a preparation containing iron hydroxide as an active ingredient as described in JP 5-155776 A. However, their phosphoric acid adsorbing action is not regarded as being sufficient.

On the other hand, reaction products between natural polysaccharide alginate having a carboxylic acid functional group and iron include swelling ferric alignate as a medicine for intestinal disorders as described in JP 51-142546 A, water-insoluble ferrous alginate as an iron supplement as described in JP 60-72817 A, and water-soluble ferric alginate as being effective for the prevention and amelioration of iron deficiency anemia as described in JP 5-244900 A. However, no mention has been made that the iron alginates have phosphoric acid adsorbing action or utility as a therapeutic agent for hyperphosphatemia.

## DISCLOSURE OF THE INVENTION

It is an object of the present invention to provide an adsorbent for phosphoric acid that solves the above-mentioned problems of the prior art, can be produced with ease, exhibits high performance for the adsorption of phosphoric acid and is free from any problems in the use thereof.

The inventors of the present invention have studied extensively with a view to achieving the above-mentioned object and as a result have found that a water-insoluble gel-like reaction product obtained by mixing a divalent or trivalent iron ion and a natural polysaccharide having a carboxylic acid functional group or a sulfuric acid functional group, such as alginate, pectin or carrageenan, in a solution has excellent phosphoric acid adsorbing characteristics, thus accomplishing an invention on a novel adsorbent for phosphoric acid. The gel-like reaction product obtained by using a trivalent iron ion shows a decrease in phosphoric acid adsorption reaction characteristics when it is dried and the inventors of the present invention have further devised an improved method for preventing the decrease in adsorbability. That is, they have found that a gel-like reaction product obtained by mixing three components, i.e., a trivalent iron ion, a natural polysaccharide having a carboxylic acid functional group or a sulfuric acid functional group, and a hydrophilic polymer such as agar in a solution does not exhibit phosphoric acid adsorbing capability even when dried into a dried product.

The present invention also relates to a method of absorbing phosphoric acid by using a reaction product between an iron ion and a natural polysaccharide having a carboxylic acid functional group or a sulfuric acid functional group. According to the present invention, it is possible to adsorb phosphoric acid.

As mentioned above, the inventors of the present invention have extensively carried out experiments on the method of preparing a novel reaction product including a natural polysaccharide having a carboxylic acid functional group or a sulfuric acid functional group and divalent or trivalent iron ion as a crosslinking agent and its function and method of using it and have found that a novel reaction product having adsorption capability for phosphoric acid can be prepared, thus completing the present invention.

The adsorbent for phosphoric acid of the present invention enables adsorption of phosphoric acid from high concentration organic solutions and high concentration salt solutions, is produced at a low cost and is safe to the environment, and requires no special tanks when using it. Further, the product of the present invention has the feature that it uses natural substances that are harmless to the human body. From these properties, the product of the present invention can be used as a medicine and a therapeutic agent for hyperphosphatemia.

The present invention provides an adsorbent for phosphoric acid, including a water-insoluble reaction product obtained by mixing divalent or trivalent iron ion and a natural polysaccharide having a carboxylic acid functional group or a sulfuric acid functional group in a solution or a dried product thereof. Further, the present invention provides an adsorbent for phosphoric acid, including a dried product of a water-insoluble reaction product obtained by mixing three components, i.e., a trivalent iron ion, a natural polysaccharide having a carboxylic acid functional group or a sulfuric acid functional group and a hydrophilic polymer in a solution.

Further, the present invention provides a dried product of a water-insoluble reaction product obtained by mixing three components, i.e., a trivalent iron ion, a natural polysaccharide having a carboxylic acid functional group or a sulfuric acid functional group and a hydrophilic polymer in a solution.

Further, the present invention provides a dried product of a water-insoluble reaction product obtained by mixing a divalent iron ion and a natural polysaccharide having a carboxylic acid functional group or a sulfuric acid functional group in a solution.

Further, the present invention provides a method of adsorbing phosphoric acid by using the above-mentioned adsorbent for phosphoric acid, water-insoluble reaction product or dried product. The present invention provides a use of the above-mentioned water-insoluble reaction product or dried product in the production of adsorbent for phosphoric acid. Further, the present invention provides the adsorbent for phosphoric acid for the therapy of hyperphosphatemia. Still further, the present invention provides a method for the therapy of hyperphosphatemia by administering a pharmacologically effective amount of the above-mentioned adsorbent for phosphoric acid, water-insoluble reaction product or dried product to a patient. Alternatively, the present invention provides a use of the above-mentioned adsorbent for phosphoric acid, water-insoluble reaction product or dried product in the production of a therapeutic agent for hyperphosphatemia.

## EMBODIMENT MODE FOR CARRYING OUT THE INVENTION

Hereinafter, the present invention will be described in detail.

A natural polysaccharide having a carboxylic acid functional group or a sulfuric acid functional group include alginate, pectin, carrageenan, hyaluronic acid, keratan sulfuric acid, chondroitin sulfate, fucoidan, and heparan sulfate. Among these, alginate, pectin, and carrageenan are preferable. Two or more of the natural polysaccharides may be used as mixtures.

Alginate is an extract form seaweeds and includes D-mannuronic acid and L-guluronic acid contained in the cell wall or intercellular interstices of seaweeds as major constituent sugars. It is a viscous polysaccharide. The alginate is not only automatically taken when people eat seaweeds but also widely used in industry in, for example, a thickener, a fixing agent, a printing agent and the like. Further, alginate is a safe natural substance which is used in the form of calcium alginate as a hydrophilic additive in a human wound covering agent, cosmetics and so forth.

Pectin is a polysaccharide that is contained in every terrestrial plants and experienced for eating since old. Pectin has a molecular structure consisting mainly of polygalacturonic acid; it has been almost established that the molecular weight of commercially available pectin is 50,000 to 150,000. Pectin contains besides polygalacturonic acid, about 20% to 25% based on the entire weight of neutral sugars such as L-rhamnose, D-galactose, D-xylose, and L-fucose on the total weight thereof.

Carrageenan is a hardly digestible polysaccharide derived from a natural seaweed (red algae), known as a sulfated polysaccharide. Actually, carrageenan is an acidic polymer consisting of galactose containing a sulfuric acid group or an anhydro group as a functional group. Its basic structure is a dimer consisting of two galactoses, and the molecule is formed by binding about 500 to 2,000 units of the dimer as a repeating unit.

The iron ion used is a divalent or trivalent iron ion; water-soluble divalent or trivalent iron salts such as ferrous chloride, ferric chloride, ferrous nitrate, ferric nitrate, ferrous sulfate, and ferric sulfate can be used. Note that iron is an essential nutrient for humans, contained as iron ion, ferro-protein and other organic iron in many foods such as meats and dairy. Further, inorganic and organic iron compounds are used as a medicine for the supply of iron.

In the step of mixing an iron ion and a natural polysaccharide having a carboxylic acid functional group or a sulfuric acid functional group in a solution to obtain a water-insoluble reaction product, it is preferable that the iron ion is used as an iron ion solution obtained by preliminarily dissolving the above-mentioned iron salts in water and the natural polysaccharide having a carboxylic acid functional group or a sulfuric acid functional group is used as a natural polysaccharide solution obtained by preliminarily dissolving it in water in the same manner. In particular, it is preferable that the mixing is performed by dripping the natural polysaccharide solution into the iron ion solution. Here, the concentration of iron ion is optional but is conveniently about 0.5% to about 8% (W/V). Also, the concentration of the natural polysaccharide solution is not particularly limited but is preferably 0.05% to 10% (W/V).

When a trivalent iron ion is used as the iron ion, drying the formed water-insoluble reaction product results in loss of the adsorbability for phosphoric acid. However, such a decrease in the activity can be avoided by addition of a hydrophilic polymer. That is, a water-insoluble reaction product obtained by mixing three components, i.e., a trivalent iron ion, a natural polysaccharide having a carboxylic acid functional group or a sulfuric acid functional group, and a hydrophilic polymer, in a solution can maintain sufficient adsorbability for phosphoric acid even when dried into a dried product. The mixing of the three components may be performed by, for example, dripping an aqueous natural polysaccharide solution containing a hydrophilic polymer obtained by preliminarily adding the hydrophilic polymer to the natural polysaccharide having a carboxylic acid functional group or a sulfuric acid functional group into an aqueous solution of the trivalent iron ion. Alternatively, the mixing may be performed by immersing a water-insoluble reaction product obtained by mixing a trivalent iron ion and the natural polysaccharide having a carboxylic acid functional group or a sulfuric acid functional group in an aqueous solution, for example, a water-insoluble reaction product obtained by dripping a natural polysaccharide solution having a carboxylic acid functional group or a sulfuric acid functional group into an aqueous trivalent iron ion solution, in an aqueous hydrophilic polymer solution. Note that the dried products are novel, which constitute a part of the present invention. Further, examples of the hydrophilic polymer may include hydrophilic polysaccharides such as agar, pullulan, xanthan gum, and guar gum, and gelatin, with agar being particularly preferable. Two or more of the hydrophilic polymers may be used as mixtures.

As mentioned above, the present invention basically relates to a reaction product of a natural polysaccharide containing an iron ion as a crosslink by utilizing the bindability of the natural polysaccharide having a carboxylic acid functional group or a sulfuric acid functional group with the iron ion; the presence of a carboxyl group or a sulfuric acid group provides strong adsorbability for phosphoric acid. Examples of such a reaction product include a reaction product of alginate containing an iron ion as a crosslink, a reaction product of pectin containing an iron ion as a crosslink, and a reaction product of carrageenan containing an iron ion as a crosslink. All of them are water-insoluble gels having strong hydrophilicity; when the concentration of the natural polysaccharide is set to 0.25% (W/V), they contain about 99% (W/W) moisture and have permeability that permits sufficient passage of molecules having a molecular weight of several thousands.

The present invention also relates to a method of adsorbing phosphoric acid characterized by using the above-mentioned adsorbent for phosphoric acid. For example, when several grams on a dry basis of the water-insoluble reaction product obtained by mixing an iron ion and a natural polysaccharide having a carboxylic acid functional group or a sulfuric acid functional group in a solution or a dried product thereof is taken and 10 ml of solution which contains about 20 ppm of phosphoric acid and in which salts such as table salt, organic substances such as albumin and glycocholic acid and the like are coexisting is added, followed by shaking for about 3 hours, only phosphoric acid can be completely removed. Thus, practicing the method of adsorbing phosphoric acid by using the adsorbent for phosphoric acid of the present invention enables complete adsorption and removal of phosphoric acid even in solutions containing large amounts of contaminants such as salts and organic substances other than phosphoric acid.

The adsorbent for phosphoric acid of the present invention can be also used in medicines such as a therapeutic agent for hyperphosphatemia. For the therapeutic agent for hyperphosphatemia, it is preferable that a dried gel product containing divalent iron ion is used. Among others, a ferrous alginate dried gel product is preferable. When the above-mentioned dried gel product is used as a therapeutic agent for hyperphosphatemia, its preparation form is not particularly limited and it can be formulated into preparations in the form that ordinary medicines, such as tablet, pill, and capsule, can take according to the known method and orally administered. The amount of active ingredient in the preparation is not particularly limited and may be selected from a wide range; usually, it is preferable that the active ingredient is contained in about 30% to about 100% (W/W) in medical preparations. The dosage of the above-mentioned medical preparations may be appropriately selected depending on the age, sex and other conditions as well as severity of the disorder and the like of patients. When the medical preparations are used as an oral adsorbent for phosphoric acid, usually the daily dosage for an adult is 0.05 g to 5 g per day as active ingredient, and can be administered once to several times a day.

Note that "phosphoric acid" as used herein refers to not only $H_3PO_4$ but also various ionic states, such as $H_2PO_4^-$, $HPO_4^{2-}$, and $PO_4^{3-}$ and is used generically as including them.

BRIEF DESCRIPTION OF THE DRAWINGS

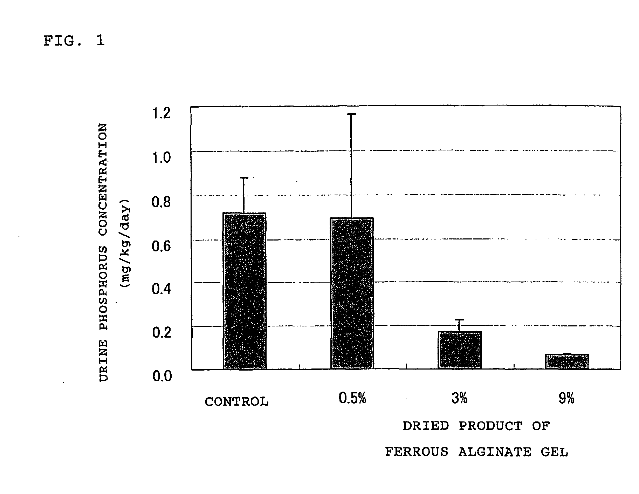

Fig. 1 is a graph illustrating a decrease in urine phosphorus excretion level in rats caused by administration of a ferrous alginate dried gel product.

EXAMPLES

Hereinafter, the present invention will be described in more detail by examples. Note that "%" about concentrations is by "W/V" unless otherwise indicated particularly.

[Production Example 1] (Production of ferric alignate gel)

200 ml of aqueous 0.25% sodium alginate solution was dripped by using a roller pump at a rate of about 1 ml/second into 500 ml of aqueous solution of 1% ferric chloride ($FeCl_3$) in a beaker with stirring. The diameter of droplets upon dripping was about 1 mm to about 2 mm. This operation formed a reaction product gel in the form of a substantially regular sphere of about 1.5 mm to about 3 mm in diameter. The gel was transferred to a 300 μm sieve and washed with pure water until the color of the aqueous iron solution is completely lost to produce 52.2 g of gel.

[Production Example 2] (Production of ferric pectin gel)

200 ml of aqueous 0.25% pectin solution was dripped by using a roller pump at a rate of about 1 ml/second into 500 ml of aqueous solution of 1% ferric chloride ($FeCl_3$) in a beaker with stirring. The diameter of droplets upon dripping was about 1 mm to about 2 mm. By this operation was formed a reaction product gel having an appearance similar to erythiocyte of about 1.5 mm to about 3 mm in diameter. The gel was transferred to a 300 μm sieve and washed with pure water until the color of the aqueous iron solution is completely lost to produce 51.3 g of gel.

[Test Example 1] (Phosphoric acid adsorption characteristics of ferric alginate gel)

About 0.2 g each of 5 lots of the reaction product gel prepared by the method of Production Example 1 were each separated in a beaker, to which 10 ml of 200 ppm phosphoric acid was added and the resultant was shaken at 37°C for 12 hours. Thereafter, the resultant was centrifuged at 3,500 rpm by using a UF filter having a molecular weight of 5,000, and then the filtrate was measured by ion chromatography to determine phosphoric acid remaining therein. The phosphoric acid adsorbability per kg of ferric alginate gel (reaction product gel) was calculated according to the following equation to obtain the results as shown in Table 1.

Mole number of phosphoric acid adsorbed by 1 kg of reaction

gel product =

(Concentration, ppm, of phosphoric acid in a sample solution

- Concentration, ppm, of phosphoric acid after application of

reaction product gel)/(100 $\times$ 95 $\times$ Weight, g, of reaction product

gel)

where 95 is the formula weight of phosphate ion $PO_4$.

Table 1

| Ferric alginate gel | | Concentration of phosphoric acid (ppm) | | Phosphoric acid adsorbability (mol/kg) |
|---|---|---|---|---|
| Production lot No. | Weight (g) | Before adsorption | After adsorption | |
| 1 | 0.21 | 200 | 190.0 | 0.0050 |
| 2 | 0.21 | | 189.4 | 0.0053 |
| 3 | 0.23 | | 188.8 | 0.0051 |
| 4 | 0.20 | | 190.5 | 0.0050 |
| 5 | 0.22 | | 188.3 | 0.0056 |
| | | | | Average 0.00520 |

The results of Table 1 indicate that the gel prepared by the method of Production Example 1 exhibited reproducibility of adsorption characteristics for phosphoric acid in a stable manner. Note that phosphoric acid remained after the adsorption by the reactionproduct gel since the tests were carried out under conditions of excessive phosphoric acid.

[Test Example 2] (Phosphoric acid adsorption characteristics of ferric pectin gel)

About 0.2 g each of 5 lots of the reaction product gel prepared by the method of Production Example 2 were each separated in a beaker, to which 10 ml of 200 ppm phosphoric acid was added and the resultant was shaken at 37°C for 12 hours. Thereafter, the resultant was centrifuged at 3,500 rpm by using a UF filter having a molecular weight of 5,000, and then the filtrate was measured by ion chromatography to determine phosphoric acid remaining therein. The phosphoric acid adsorbability per kg of ferric pectin gel was calculated according to the same equation as in Test Example 1 to obtain the results as shown in Table 2.

Table 2

| Ferric pectin gel | | Concentration of phosphoric acid (ppm) | | phosphoric acid adsorbability (mol/kg) |
|---|---|---|---|---|
| Production lot No. | Weight (g) | Before adsorption | After adsorption | |
| 1 | 0.21 | 200 | 190.5 | 0.0048 |
| 2 | 0.20 | | 191.1 | 0.0047 |
| 3 | 0.21 | | 190.1 | 0.0049 |
| 4 | 0.20 | | 191.2 | 0.0046 |
| 5 | 0.21 | | 190.4 | 0.0048 |
| | | | | Average 0.00476 |

The results of Table 2 indicate that the gel prepared by the method of Production Example 2 exhibited reproducibility of adsorption characteristics for phosphoric acid in a stable manner.

[Test Example 3] (Phosphoric acid adsorption characteristics of ferric alginate gel aqueous solution containing phosphoric acid and other salts)

About 0.2 g of the reaction product gel prepared by the method of Production Example 1 was taken in a beaker, to which 10 ml of aqueous solution containing 200 ppm phosphoric acid, 1, 000 ppm table salt, and 1,000 ppm sodium sulfate was added, and the resultant was shaken at 37°C for 12 hours. Thereafter, the resultant was centrifuged at 3,500 rpm by using a UF filter having a molecular weight of 5,000, and then the filtrate was measured by ion chromatography to determine phosphoric acid remaining therein. The phosphoric acid adsorbability per kg of ferric alginate gel was calculated according to the same equation as in Test Example 1 to obtain the results as shown in Table 3.

Table 3

| Ferric alginate gel | | Concentration of phosphoric acid (ppm) | | Phosphoric acid adsorbability (mol/kg) |
|---|---|---|---|---|
| Production lot No. | Weight (g) | Before adsorption | After adsorption | |
| 1 | 0.22 | 200 | 189.2 | 0.0052 |
| 2 | 0.23 | | 188.5 | 0.0053 |
| 3 | 0.21 | | 189.9 | 0.0051 |
| 4 | 0.22 | | 188.5 | 0.0055 |
| 5 | 0.20 | | 190.3 | 0.0051 |
| | | | | Average 0.00524 |

The results shown in Table 3 indicate that the gel prepared in Production Example 1 held the phosphoric acid adsorbability even when high concentrations of salts such as table salt and sodium sulfate are contained. Further, consideration taking the test results of Test Example 1 in combination indicates that the phosphoric acid adsorbability did not decrease in spite of high concentrations of salts such as table salt and sodium sulfate.

[Test Example 4] (Phosphoric acid adsorption characteristics of ferric pectin gel aqueous solution containing phosphoric acid and other salts)

About 0.2 g of the reaction product gel prepared by the method of Production Example 2 was separated in a beaker, to which 10 ml of aqueous solution containing 200 ppm phosphoric acid, 1,000 ppm table salt, and 1, 000 ppm sodium sulfate was added, and the resultant was shaken at 37°C for 12 hours. Thereafter, the resultant was centrifuged at 3,500 rpm by using a UF filter having a molecular weight of 5,000, and then the filtrate was measured by ion chromatography to determine phosphoric acid remaining therein. The phosphoric acid adsorbability per kg of ferric pectin gel was calculated according to the same equation as in Test Example 1 to obtain the results as shown in Table 4.

Table 4

| Ferric pectin gel | | Concentration of phosphoric acid (ppm) | | Phosphoric acid adsorbability (mol/kg) |
|---|---|---|---|---|
| Production lot No. | Weight (g) | Before adsorption | After adsorption | |
| 1 | 0.20 | 200 | 190.9 | 0.0048 |
| 2 | 0.20 | | 191.0 | 0.0047 |
| 3 | 0.21 | | 190.8 | 0.0046 |
| 4 | 0.21 | | 191.5 | 0.0043 |
| 5 | 0.20 | | 190.7 | 0.0049 |
| | | | | Average 0.00466 |

The results shown in Table 4 indicate that the gel prepared in Production Example 2 held the phosphoric acid adsorbability even when high concentrations of salts such as table salt and sodium sulfate are contained. Further, consideration taking the test results of Test Example 2 in combination indicates that the phosphoric acid adsorbability did not decrease in spite of high concentrations of salts such as table salt and sodium sulfate.

[Test Example 5] (Phosphoric acid adsorption characteristics of ferric alginate gel in a phosphoric acid solution containing high concentration table salt and organic substance)

About 0.2 g of the reaction product gel prepared by the method of Production Example 1 was separated in a beaker, to which 10 ml of phosphoric acid solution obtained by adding phosphoric acid to a solution containing the components shown in Table 5 given below to a concentration of 200 ppm and adjusting pH to 7.5 with 0.1 M-HCl and 0.1 M-NaOH was added, and the resultant was shaken at 37°C for 12 hours. Thereafter, the resultant was centrifuged at 3,500 rpm by using a UF filter having a molecular weight of 5,000, and then the filtrate was measured by ion chromatography to determine phosphoric acid remaining therein. The phosphoric acid adsorbability per kg of ferric alginate gel was calculated according to the same equation as in Test Example 1 to obtain the results as shown in Table 6.

Table 5

| Ammonia | 2mg/dl (28%$NH_4OH$ 0.07g/L) |
|---|---|
| Urea | 100mg/dl (1.0g/L) |
| NaCl | 500mg/dl (5.0g/L) |
| Albumin | 400mg/dl (4.0g/L) |
| Glycocholic acid (abt. MW400) | 0.2mM/dl (0.8g/L) |

Table 6

| Ferric alginate gel | | Phosphoric acid concentration (ppm) | | Phosphoric acid adsorbability (mol/kg) |
|---|---|---|---|---|
| Production lot No. | Weight (g) | Before adsorption | After adsorption | |
| 1 | 0.20 | 200 | 190.9 | 0.0048 |
| 2 | 0.20 | | 189.9 | 0.0053 |
| 3 | 0.21 | | 189.1 | 0.0055 |
| 4 | 0.22 | | 188.8 | 0.0054 |
| 5 | 0.19 | | 190.5 | 0.0053 |
| | | | | Average 0.00526 |

Comparison of the results shown in Table 6 given above with those of Test Example 1 and Test Example 3 indicates that the gel prepared in Production Example 1 did not show a decrease in adsorption characteristics for phosphoric acid in an aqueous solution containing high concentrations of salts such as table salt and sodium sulfate.

[Test Example 6] (Phosphoric acid adsorption characteristics of ferric pectin gel in a phosphoric acid solution containing high concentration table salt and organic substance)

About 0.2 g of the reaction product gel prepared by the method of Production Example 2 was separated in a beaker, to which 10 ml of phosphoric acid solution obtained by adding phosphoric acid to a solution containing the components shown in Table 5 given above to a concentration of 200 ppm and adjusting pH to 7.5 with 0.1 M-HCl and 0.1 M-NaOH was added, and the resultant was shaken at 37°C for 12 hours. Thereafter, the resultant was centrifuged at 3,500 rpm by using a UF filter having a molecular weight of 5,000, and then the filtrate was measured by ion chromatography to determine phosphoric acid remaining therein. The phosphoric acid adsorbability per kg of ferric pectin gel was calculated according to the same equation as in Test Example 1 to obtain the results as shown in Table 7.

Table 7

| Ferric pectin gel | | Concentration of phosphoric acid (ppm) | | Phosphoric acid adsorbability (mol/kg) |
|---|---|---|---|---|
| Production lot No. | Weight (g) | Before adsorption | After adsorption | |
| 1 | 0.20 | 200 | 190.7 | 0.0049 |
| 2 | 0.21 | | 191.3 | 0.0044 |
| 3 | 0.20 | | 190.9 | 0.0048 |
| 4 | 0.21 | | 191.6 | 0.0047 |
| 5 | 0.21 | | 190.4 | 0.0048 |
| | | | | Average 0.00472 |

Comparison of the results shown in Table 7 given above with those of Test Example 2 and Test Example 4 indicates that the gel prepared in Production Example 2 did not show a decrease in adsorption characteristics for phosphoric acid in an aqueous solution containing high concentrations of salts such as table salt and sodium sulfate.

[Production Example 3] (Production of dried product of agar-immersed ferric alginate gel)

52.2 g of the ferric alginate gel prepared in Production Example 1 was charged in 1,000 ml of 0.1% agar solution to immerse for 1 hour while stirring and the resultant was transferred to an about 300 μm sieve and washed with 20 liters of pure water at about 60°C, followed by drying the resultant in an incubator at 60°C for 5 hours to prepare 0.53 g of a dried product of agar-immersed ferric alginate gel.

[Production Example 4] (Production of dried product of agar-immersed ferric pectin gel)

51.3 g of the ferric alginate gel prepared in Production Example 2 was charged in 1,000 ml of 0.1% agar solution to immerse for 1 hour while stirring and the resultant was transferred to an about 300 μm sieve and washed with 20 liters of pure water at about 60°C, followed by drying the resultant in an incubator at 60°C for 5 hours to prepare 0.52 g of a dried product of agar-immersed ferric pectin gel.

[Test Example 7] (Adsorption characteristics of dried product of agar-immersed ferric alginate gel for phosphoric acid)

20 mg of the dried product of agar-immersed ferric alginate gel prepared in Production Example 3 was separated in a beaker, to which 10 ml of 200 ppm phosphoric acid was added and the resultant was shaken at 37°C for 12 hours. Thereafter, the resultant was centrifuged at 3,500 rpm by using a UF filter having a molecular weight of 5,000, and then the filtrate was measured by ion chromatography to determine phosphoric acid remaining therein. In addition, for comparison, similar tests were carried out for the ferric alginate gel prepared in Production Example 1 and dried in an incubator at 60°C for 5 hours. The phosphoric acid adsorbability per kg of a dried gel product was calculated according to the following equation to obtain the results as shown in Table 8.

Mole number of phosphoric acid adsorbed by 1 kg of a dried

gel product =

(Concentration, ppm, of phosphoric acid in a sample solution

- Concentration, ppm, of phosphoric acid after application of a

dried gel product)/(100 $\times$ 95 $\times$ Weight, g, of a dried gel product)

where 95 is the formula weight of phosphate ion $PO_4$.

Table 8

| Dried product of agar-immersed ferric alginate gel | | Concentration of phosphoric acid (ppm) | | Phosphoric acid adsorbability (mol/kg) |
|---|---|---|---|---|
| Production lot No. | Weight (mg) | Before adsorption | After adsorption | |
| 1 | 21 | 200 | 110.0 | 0.45 |
| 2 | 21 | | 112.3 | 0.44 |
| 3 | 20 | | 120.6 | 0.42 |
| 4 | 21 | | 115.2 | 0.43 |
| 5 | 20 | | 118.2 | 0.43 |
| | | | | Average 0.434 |
| Dried product obtained by drying preparation of Example 1 (20mg) | | 200 | 192.3 | 0.04 |

The results of Table 8 indicate that the dried product of agar-immersed ferric alginate gel prepared by the method of Production Example 3 held phosphoric acid adsorbability in contrast to the ferric alginate gel of Production Example 1 which lost the phosphoric acid adsorbability by drying.

[Test Example 8] (Adsorption characteristics of dried product of agar-immersed ferric pectin gel for phosphoric acid)

20 mg of the dried product of agar-immersed ferric pectin gel prepared in Production Example 4 was separated in a beaker, to which 10 ml of 200 ppm phosphoric acid was added and the resultant was shaken at 37°C for 12 hours. Thereafter, the resultant was centrifuged at 3,500 rpm by using a UF filter having a molecular weight of 5,000, and then the filtrate was measured by ion chromatography to determine phosphoric acid remaining therein. In addition, for comparison, similar tests were carried out for the ferric pectin gel prepared in Production Example 2 and dried in an incubator at 60°C for 5 hours. The phosphoric acid adsorbability per kg of a dried gel product was calculated according to the same equation as in Test Example 7 to obtain the results as shown in Table 9.

Table 9

| Dried product of agar-immersed ferric pectin gel | | Concentration of phosphoric acid (ppm) | | Phosphoric acid adsorbability (mol/kg) |
|---|---|---|---|---|
| Production lot No. | Weight (mg) | Before adsorption | After adsorption | |
| 1 | 20 | 200 | 126.0 | 0.39 |
| 2 | 19 | | 127.2 | 0.40 |
| 3 | 21 | | 122.1 | 0.39 |
| 4 | 20 | | 122.6 | 0.41 |
| 5 | 21 | | 121.3 | 0.39 |
| | | | | Average 0.396 |
| Dried product obtained by drying preparation of Example 2 (20mg) | | 200 | 152.5 | 0.25 |

The results of Table 9 indicate that the dried product of agar-immersed ferric pectin gel prepared by the method of Production Example 4 held phosphoric acid adsorbability in contrast to the ferric pectin gel of Production Example 2 which lost the phosphoric acid adsorbability by drying.

[Test Example 9] (Adsorption characteristics of dried product of agar- immersed ferric alginate gel for phosphoric acid in a phosphoric acid solution containing high concentration table salt and organic substance)

20 mg of the dried product of agar-immersed ferric alginate gel prepared in Production Example 3 was separated in a beaker, to which 10 ml of phosphoric acid solution obtained by adding phosphoric acid to a solution containing the components shown in Table 5 given above to a concentration of 200 ppm and adjusting pH to 7.5 with 0.1 M-HCl and 0.1 M-NaOH was added, and the resultant was shaken at 37°C for 12 hours. Thereafter, the resultant was centrifuged at 3,500 rpm by using a UF filter having a molecular weight of 5,000, and then the filtrate was measured by ion chromatography to determine phosphoric acid remaining therein. In addition, for comparison, similar tests were carried out for the ferric alginate gel prepared in Production Example 1 and dried in an incubator at 60°C for 5 hours. The phosphoric acid adsorbability per kg of a dried gel product was calculated according to the same equation as in Test Example 7 to obtain the results as shown in Table 10.

Table 10

| Dried product of agar-immersed ferric alginate gel | | Concentration of phosphoric acid (ppm) | | Phosphoric acid adsorbability (mol/kg) |
|---|---|---|---|---|
| Production lot No. | Weight (mg) | Before adsorption | After adsorption | |
| 1 | 20 | 200 | 116.2 | 0.44 |
| 2 | 20 | | 118.1 | 0.43 |
| 3 | 21 | | 115.4 | 0.42 |
| 4 | 22 | | 104.5 | 0.46 |
| 5 | 19 | | 120.1 | 0.44 |
| | | | | Average 0.438 |
| Dried product obtained by drying preparation of Example 1 (20mg) | | 200 | 193.3 | 0.04 |

The results shown in Table 10 indicate that the dried product of agar-immersed ferric alginate gel repeatedly held clear and stable phosphoric acid adsorbability even when high concentrations of salts and organic substance are contained.

[Test Example 10] (Adsorption characteristics of dried product of agar-immersed ferric pectin gel for phosphoric acid in a phosphoric acid solution containing high concentration table salt and organic substance)

20 mg of the dried product of agar-immersed ferric pectin gel prepared in Production Example 4 was separated in a beaker, to which 10 ml of phosphoric acid solution obtained by adding phosphoric acid to a solution containing the components shown in Table 5 given above to a concentration of 200 ppm and adjusting pH to 7.5 with 0.1 M-HCl and 0.1 M-NaOH was added, and the resultant was shaken at 37°C for 12 hours. Thereafter, the resultant was centrifuged at 3,500 rpm by using a UF filter having a molecular weight of 5, 000, and then the filtrate was measured by ion chromatography to determine phosphoric acid remaining therein. In addition, for comparison, similar tests were carried out for the ferric pectin gel prepared in Example 2 and dried in an incubator at 60°C for 5 hours. The phosphoric acid adsorbability per kg of a dried gel product was calculated according to the same equation as in Test Example 7 to obtain the results as shown in Table 11.

Table 11

| Dried product of agar-immersed ferric pectin gel | | Concentration of phosphoric acid (ppm) | | Phosphoric acid adsorbability (mol/kg) |
|---|---|---|---|---|
| Production lot No. | Weight (mg) | Before adsorption | After adsorption | |
| 1 | 20 | 200 | 122.4 | 0.41 |
| 2 | 20 | | 123.2 | 0.40 |
| 3 | 19 | | 129.5 | 0.39 |
| 4 | 20 | | 123.7 | 0.40 |
| 5 | 20 | | 124.1 | 0.40 |
| | | | | Average 0.400 |
| Dried product obtained by drying preparation of Example 2 (21mg) | | 200 | 155.3 | 0.22 |

The results shown in Table 11 indicate that the dried product of agar-immersed ferric pectin gel repeatedly held clear and stable phosphoric acid adsorbability even when high concentrations of salts and organic substance are contained.

[Production Example 5] (Production of dried product of ferric alginate-agar gel)

200 ml of mixed solution prepared by adding agar to a 0.25% sodium alginate solution to a concentration of 0.1% was dripped by using a roller pump at a rate of about 1 ml/second into 500 ml of 1% ferric chloride (FeCl$_3$) solution in a beaker with stirring. The diameter of droplets upon dripping was about 1 mm to about 2 mm. This operation formed a reaction product gel in the form of a substantially regular sphere of about 1.5 mm to about 3 mm in diameter. The gel was transferred to an about 300 µm sieve and washed with pure water until the color of the aqueous iron solution is completely lost, followed by drying the resultant in an incubator at 60°C for 5 hours to prepare 0.50 g of a dried product of ferric alginate-agar gel.

[Production Example 6] (Production of dried product of ferric pectin-agar gel)

200 ml of mixed solution prepared by adding agar to an aqueous 0.25% pectin solution to a concentration of 0.1% was dripped by using a roller pump at a rate of about 1 ml/second into 500 ml of aqueous solution of 1% ferric chloride (FeCl$_3$) in a beaker with stirring. The diameter of droplets upon dripping was about 1 mm to about 2 mm. This operation formed a reaction product gel having an external appearance similar to an erythrocyte of about 1.5 mm to about 3 mm in diameter. The gel was transferred to an about 300 µm sieve and washed with pure water until the color of the aqueous iron solution is completely lost, followed by drying the resultant in an incubator at 60°C for 5 hours to prepare 0.49 g of a dried product of ferric pectin-agar gel.

[Test Example 11] (Adsorption characteristics of phosphoric acid by a dried product of ferric alginate-agar gel)

20 mg of the dried product of ferric alginate-agar gel prepared in Production Example 5 was separated in a beaker, to which 10 ml of 200 ppm phosphoric acid was added and the resultant was shaken at 37°C for 12 hours. Thereafter, the resultant was centrifuged at 3, 500 rpm by using a UF filter having a molecular weight of 5, 000, and then the filtrate was measured by ion chromatography to determine phosphoric acid remaining therein. In addition, for comparison, similar tests were carried out for the alginate gel prepared in Example 1 and dried in an incubator at 60°C for 5 hours. The phosphoric acid adsorbability per kg of a dried gel product was calculated according to the same equation as in Test Example 7 to obtain the results as shown in Table 12.

Table 12

| Dried product of ferric alginate-agar gel | | Phosphoric acid concentration (ppm) | | Phosphoric acid adsorbability (mol/kg) |
|---|---|---|---|---|
| Production lot No. | Weight (mg) | Before adsorption | After adsorption | |
| 1 | 22 | 200 | 111.5 | 0.42 |
| 2 | 21 | | 112.3 | 0.44 |
| 3 | 19 | | 114.2 | 0.43 |
| 4 | 21 | | 107.7 | 0.46 |
| 5 | 21 | | 111.5 | 0.44 |
| | | | | Average 0.438 |
| Dried product obtained by drying preparation of Example 1 (21mg) | | 200 | 194.4 | 0.028 |

The results shown in Table 12 indicate that the dried product of ferric alginate-agar gel produced in Production Example 5 repeatedly exhibited clear and stable phosphoric acid adsorbability so that it could avoid a decrease in phosphoric acid adsorbability due to drying in contrast to the ferric alginate reaction product in Production Example 1 which showed a decrease in phosphoric acid adsorbability due to drying.

[Test Example 12] (Adsorption characteristics of dried product of ferric pectin-agar gel for phosphoric acid)

About 20 mg of the dried product of ferric pectin-agar gel prepared in Production Example 6 was separated in a beaker, to which 10 ml of an aqueous 200 ppm phosphoric acid solution was added and the resultant was shaken at 37°C for 12 hours. Thereafter, the resultant was centrifuged at 3,500 rpm by using a UF filter having a molecular weight of 5,000, and then the filtrate was measured by ion chromatography to determine phosphoric acid remaining therein. In addition, for comparison, similar tests were carried out for the ferric pectin gel prepared in Example 2 and dried in an incubator at 60°C for 5 hours. The phosphoric acid adsorbability per kg of a dried gel product was calculated according to the same equation as in Test Example 7 to obtain the following results.

Table 13

| Dried product of ferric pectin-agar gel | | Phosphoric acid concentration (ppm) | | phosphoric acid adsorbability (mol/kg) |
|---|---|---|---|---|
| Production lot No. | Weight (mg) | Before adsorption | After adsorption | |
| 1 | 21 | 200 | 121.5 | 0.39 |
| 2 | 20 | | 125.1 | 0.39 |
| 3 | 19 | | 126.1 | 0.41 |
| 4 | 21 | | 121.3 | 0.39 |
| 5 | 21 | | 119.2 | 0.41 |
| | | | | Average 0.395 |
| Dried product obtained by drying preparation of Example 2 (20mg) | | 200 | 165.6 | 0.18 |

The results shown in Table 13 indicate that the dried product of ferric pectin-agar gel produced in Production Example 6 repeatedly exhibited clear and stable phosphoric acid adsorbability so that it could avoid a decrease in phosphoric acid adsorbability due to drying in contrast to the ferric pectin reaction product in Production Example 2 which showed a decrease in phosphoric acid adsorbability due to drying.

[Test Example 13] (Adsorption characteristics of dried product of ferric alginate-agar gel for phosphoric acid in a phosphoric acid solution containing high concentration table salt and organic substance)

About 20 mg of the dried product of ferric alginate-agar gel prepared in Production Example 5 was separated in a beaker, to which 10 ml of phosphoric acid solution obtained by adding phosphoric acid to a solution containing the components shown in Table 5 given above to a concentration of 200 ppm and adjusting pH to 7.5 with 0.1 M-HCl and 0.1 M-NaOH was added, and the resultant was shaken at 37°C for 12 hours. Thereafter, the resultant was centrifuged at 3, 500 rpm by using a UF filter having a molecular weight of 5, 000, and then the filtrate was measured by ion chromatography to determine phosphoric acid remaining therein. In addition, for comparison, similar tests were carried out for the ferric alginate gel prepared in Production Example 1 and dried in an incubator at 60°C for 5 hours. The phosphoric acid adsorbability per kg of a dried gel product was calculated according to the same equation as in Test Example 7 to obtain the results as shown in Table 14.

Table 14

| Dried product of ferric alginate-agar gel | | Phosphoric acid concentration (ppm) | | Phosphoric acid adsorbability (mol/kg) |
|---|---|---|---|---|
| Production lot | Weight (mg) | Before adsorption | After adsorption | |
| 1 | 19 | 200 | 121.1 | 0.44 |
| 2 | 21 | | 104.5 | 0.48 |
| 3 | 20 | | 120.4 | 0.42 |
| 4 | 22 | | 99.7 | 0.48 |
| 5 | 20 | | 119.9 | 0.42 |

Table 14   (continued)

| Dried product of ferric alginate-agar gel | | Phosphoric acid concentration (ppm) | | Phosphoric acid adsorbability (mol/kg) |
|---|---|---|---|---|
| Production lot | Weight (mg) | Before adsorption | After adsorption | |
| | | | | Average 0.448 |
| Dried product obtained by drying preparation of Example 1 (20mg) | | 200 | 193.7 | 0.033 |

The results shown in Table 14 indicate with reproducibility that the dried product of ferric alginate-agar gel prepared in Production Example 5 had phosphoric acid adsorbability even when high concentrations of salts and organic substance coexisted. This shows that the dried product of ferric alginate-agar gel prepared in Production Example 5 could avoid a decrease in phosphoric acid adsorbability due to drying in contrast to the ferric alginate gel in Production Example 1 which showed a decrease in phosphoric acid adsorbability due to drying.

[Test Example 14] (Adsorption characteristics of dried product of ferric pectin-agar gel for phosphoric acid in a phosphoric acid solution containing high concentration table salt and organic substance)

About 20 mg of the dried product of ferric pectin-agar gel prepared in Production Example 6 was separated in a beaker, to which 10 ml of phosphoric acid solution obtained by adding phosphoric acid to a solution containing the components shown in Table 5 given above to a concentration of 200 ppm and adjusting pH to 7.5 with 0.1 M-HCl and 0.1 M-NaOH was added, and the resultant was shaken at 37°C for 12 hours. Thereafter, the resultant was centrifuged at 3, 500 rpm by using a UF filter having a molecular weight of 5, 000, and then the filtrate was measured by ion chromatography to determine phosphoric acid remaining therein. In addition, for comparison, similar tests were carried out for the gel prepared in Example 2 and dried in an incubator at 60°C for 5 hours. The phosphoric acid adsorbability per kg of a dried gel product was calculated according to the same equation as in Test Example 7 to obtain the results as shown in Table 15.

Table 15

| Dried product of ferric pectin-agar gel | | Phosphoric acid concentration (ppm) | | Phosphoric acid adsorbability (mol/kg) |
|---|---|---|---|---|
| Production lot No. | Weight (mg) | Before adsorption | After adsorption | |
| 1 | 20 | 200 | 125.2 | 0.39 |
| 2 | 21 | | 121.1 | 0.40 |
| 3 | 21 | | 120.8 | 0.40 |
| 4 | 21 | | 122.0 | 0.39 |
| 5 | 20 | | 121.0 | 0.41 |
| | | | | Average 0.398 |
| Dried product obtained by drying preparation of Example 2 (20mg) | | 200 | 162.4 | 0.20 |

The results shown in Table 15 indicate with reproducibility that the dried product of ferric pectin-agar gel prepared in Production Example 6 had phosphoric acid adsorbability even when high concentrations of salts and organic substance coexisted. This shows that the dried product of ferric pectin-agar gel prepared in Production Example 6 could avoid a decrease in phosphoric acid adsorbability due to drying in contrast to the ferric pectin gel in Production Example 2 which showed a decrease in phosphoric acid adsorbability due to drying.

[Production Example 7] (production of a dried product of ferrous alginate gel)

200 ml of 0.25% sodium alginate solution was dripped by using a roller pump at a rate of about 1 ml/second into 500 ml of 1% ferrous sulfate solution in a beaker with stirring. The diameter of droplets upon dripping was about 1 mm to about 2 mm. This operation formed a reaction product gel in the form of a substantially regular sphere of about 1.5 mm to about 3 mm in diameter. The gel was transferred to an about 100 μm sieve and washed with pure water until the concentration in water of iron ions became 1 ppm or less. Further, drying treatment was performed in an incubator at 60°C for 5 hours to prepare 0.45 g of a dry product of ferrous alginate gel.

[Production Example 8] (Production of dried product of ferrous pectin gel)

200 ml of aqueous 0.25% pectin solution was dripped by using a roller pump at a rate of about 1 ml/second into 500 ml of 1% ferrous sulfate solution in a beaker with stirring. The diameter of droplets upon dripping was about 1 mm to about 2 mm. This operation formed a reaction product gel having an irregular external appearance of about 1.5 mm to about 3 mm in diameter. The gel was transferred to an about 100 μm sieve and washed with pure water until the concentration in water of iron ions became 1 ppm or less. Further, drying treatment was performed in an incubator at 60°C for 5 hours to prepare 0.42 g of a dry product of ferrous pectin gel.

[Production Example 9] (Production of dried product of ferrous carrageenan gel)

200 ml of 0.25% carrageenan solution was dripped by using a roller pump at a rate of about 1 ml/second into 500 ml of 1% ferrous sulfate solution in a beaker with stirring. The diameter of droplets upon dripping was about 1 mm to about 2 mm. This operation formed a reaction product gel having an irregular external appearance of about 1.5 mm to about 3 mm in diameter. The gel was transferred to an about 50 μm sieve and washed with pure water until the concentration in water of iron ions became 1 ppm or less. Further, drying treatment was performed in an incubator at 60°C for 5 hours to prepare 0.40 g of a dry product of ferrous carrageenan gel.

[Test Example 15] (Phosphoric acid adsorption characteristics of dry product of ferrous alginate gel)

Pure water was added to 0.48 ml of 85% (W/W) phosphoric acid, 3.18 g of sodium carbonate and 4.68 g of sodium chloride to make 1 liter. This was named as Test Solution 1. The molar concentration of phosphoric acid in the Test Solution 1 was determined by ion chromatography. Further, about 0.1 g each of 5 lots of the dried gel product prepared by the method of Production Example 7 was separated in a beaker, to which 20 ml of the Test Solution 1 was added and the resultant was stirred at 37°C for 1 hour. Thereafter, the resultant was centrifuged at 3,500 rpm by using a UF filter having a molecular weight of 5, 000, and then the filtrate was measured by ion chromatography to determine phosphoric acid remaining therein. The phosphoric acid adsorbability per kg of a dried gel product was calculated according to the following equation to obtain the results as shown in Table 16.

Mole number of phosphoric acid adsorbed by 1 kg of a dried

gel product =

(Concentration, mmol, of phosphoric acid before application

of a dried gel product - Concentration, mmol, of phosphoric acid

after application of a dried gel product) $\times$ {20 /(Weight, g, of a

dried gel product)} ÷ 1000

Table 16

| Dried product of ferrous alginate gel | | Phosphoric acid concentration (mmol/L) | | Phosphoric acid adsorbability (mol/kg) |
|---|---|---|---|---|
| Production lot No. | Weight (g) | Before adsorption | After adsorption | |
| 1 | 0.102 | 7.02 | 0.072 | 1.362 |
| 2 | 0.094 | | 0.080 | 1.477 |
| 3 | 0.108 | | 0.080 | 1.285 |
| 4 | 0.105 | | 0.068 | 1.324 |
| 5 | 0.105 | | 0.074 | 1.323 |
| | | | | Average 1.354 |

The results of Table 16 indicate with reproducibility that the dried product of ferrous alginate gel prepared by the method of Production Example 7 had phosphoric acid adsorbability even in a system in which a carbonate ion and a chloride ion coexisted.

[Test Example 16] (Phosphoric acid adsorbability of dry product of ferrous pectin gel)

About 0.1 g each of 5 lots of the dried gel product prepared by the method of Production Example 8 was separated in a beaker, to which 20 ml of the Test Solution 1 in Test Example 15 was added and the resultant was stirred at 37°C for 1 hour. Thereafter, the resultant was centrifuged at 3,500 rpm by using a UF filter having a molecular weight of 5,000, and then the filtrate was measured by ion chromatography to determine phosphoric acid remaining therein. The phosphoric acid adsorbability per kg of a dried gel product was calculated according to the same equation as that in Test Example 15 to obtain the results as shown in Table 17.

Table 17

| Dried product of ferrous pectin gel | | Phosphoric acid concentration (mmol/L) | | Phosphoric acid adsorbability (mol/kg) |
|---|---|---|---|---|
| Production lot No. | Weight (g) | Before adsorption | After adsorption | |
| 1 | 0.104 | 7.02 | 0.957 | 1.166 |
| 2 | 0.114 | | 0.580 | 1.123 |
| 3 | 0.105 | | 0.929 | 1.160 |
| 4 | 0.104 | | 0.998 | 1.158 |
| 5 | 0.107 | | 0.896 | 1.145 |
| | | | | Average 1.150 |

The results of Table 17 indicate with reproducibility that the dried gel product prepared by the method of Production Example 8 had phosphoric acid adsorbability even in a system in which a carbonate ion and a chloride ion coexisted.

[Test Example 17] (Phosphoric acid adsorbability of dry product of ferrous carrageenan gel)

About 0.1 g each of 5 lots of the dried gel product prepared by the method of Production Example 9 was separated in a beaker, to which 20 ml of the Test Solution 1 in Test Example 15 was added and the resultant was stirred at 37°C for 1 hour. Thereafter, the resultant was centrifuged at 3,500 rpm by using a UF filter having a molecular weight of 5,000, and then the filtrate was measured by ion chromatography to determine phosphoric acid remaining therein. The phosphoric acid adsorbability per kg of a dried gel product was calculated according to the same equation as that in Test Example 15 to obtain the results as shown in Table 18.

Table 18

| Dried product of ferrous carrageenan gel | | Phosphoric acid concentration (mmol/L) | | Phosphoric acid adsorbability (mol/kg) |
|---|---|---|---|---|
| Production lot No. | Weight (g) | Before adsorption | After adsorption | |
| 1 | 0.098 | 7.02 | 5.334 | 0.344 |
| 2 | 0.102 | | 4.988 | 0.372 |
| 3 | 0.100 | | 5.070 | 0.390 |
| 4 | 0.107 | | 5.013 | 0.375 |
| 5 | 0.095 | | 5.195 | 0.384 |
| | | | | Average 0.373 |

The results of Table 18 indicate with reproducibility that the dried gel product prepared by the method of Production Example 9 had phosphoric acid adsorbability even in a system in which a carbonate ion and a chloride ion coexisted.

[Test Example 18] (Influence of dried product of ferrous alginate gel on urine phosphorus excretion in normal rats)

Evaluation was made in a test system in which female Slc:SD rats (6 weeks old) were administered with the dried gel product prepared by the method of Production Example 7. Administration of the chemical was performed by administering mixed diets prepared by mixing powder diet CRF-1 manufactured by Oriental Yeast Industry Co., Ltd. with the dried gel product in concentrations of 0.5%, 3%, and 9%, respectively, for 4 days. Urines before the administration and 4 days after the administration were collected and the concentration of phosphoric acid in the urines was determined (PNP-XDH method, HITACHI 7170S Type Auto Analyzer). Each group subjected to the experiment consisted of 6 rats.

The results obtained are shown in Table 19 and Fig. 1 (average value ± standard error). A dose-dependent decrease in urine phosphorus concentration was observed in the group administered with the dried gel product and a decrease was observed from the 3%-administered group in comparison with the control. The instant test results indicate that the dried product of ferrous alginate gel inhibits absorption of phosphoric acid through the digestive tract as a result of adsorption of phosphoric acid thereon, thus showing the effectiveness of the product of the present invention as a therapeutic agent for hyperphosphatemia.

Table 19

| Group administered | | Urine phosphorus concentration (mg/kg/day) | |
|---|---|---|---|
| | | Average value | Standard error |
| Control | | 0.7182 | 0.1598 |
| Dried product of ferrous alginate gel | 0.5% | 0.6914 | 0.4748 |
| | 3% | 0.1727 | 0.0538 |
| | 9% | 0.0633 | 0.0085 |

**Claims**

1.   An adsorbent forphosphoric acid, comprising a water-insoluble reaction product obtained by mixing a divalent or trivalent iron ion and a natural polysaccharide having a carboxylic acid functional group or a sulfuric acid functional group in a solution or a dried product thereof.

2.   The adsorbent for phosphoric acid according to claim 1, wherein the mixing is performed by converting the iron ion to an iron ion solution and the natural polysaccharide to a natural polysaccharide solution, and dripping the natural polysaccharide solution into the iron ion solution.

3.   An adsorbent for phosphoric acid, comprising a dried product of a water-insoluble reaction product obtained by mixing three components, i.e., a trivalent iron ion, a natural polysaccharide having a carboxylic acid functional group or a sulfuric acid functional group and a hydrophilic polymer in a solution.

4. The adsorbent for phosphoric acid according to claim 3, wherein the mixing of the three components is performed by dripping a natural polysaccharide solution having a carboxylic acid functional group or a sulfuric acid functional group solution containing the hydrophilic polymer into a trivalent iron ion solution.

5. The adsorbent for phosphoric acid according to claim 3, wherein the mixing of the three components is performed by immersing the water-insoluble reaction product obtained by mixing the trivalent iron ion and the natural polysaccharide having a carboxylic acid functional group or a sulfuric acid functional group in a solution in a hydrophilic polymer solution.

6. The adsorbent for phosphoric acid according to any one of claims 3 to 5, wherein the hydrophilic polymer is agar.

7. The adsorbent for phosphoric acid according to anyone of claims 1 to 6, wherein the natural polysaccharide having a carboxylic acid functional group or a sulfuric acid functional group is at least one natural polysaccharide selected from the group consisting of alignate, pectin, and carrageenan.

8. The adsorbent for phosphoric acid according to any one of claims 1 to 7 for the therapy of hyperphosphatemia.

9. A dried product of a water-insoluble reaction product obtained by mixing three components, i.e., a trivalent iron ion, a natural polysaccharide having a carboxylic acid functional group or a sulfuric acid functional group and a hydrophilic polymer in a solution.

10. The dried product according to claim 9, wherein the mixing of the three components is performed by dripping a natural polysaccharide solution having a carboxylic acid functional group or a sulfuric acid functional group containing the hydrophilic polymer into a trivalent iron ion solution.

11. The according to claim 9, wherein the mixing of the three components is performed by immersing the water-insoluble reaction product obtained by mixing the trivalent iron ion and the natural polysaccharide having a carboxylic acid functional group or a sulfuric acid functional group in a solution in a hydrophilic polymer solution.

12. The dried product according to any one of claims 9 to 11, wherein agar is used as the hydrophilic polymer.

13. The dried product according to any one of claims 9 to 12, wherein the natural polysaccharide having a carboxylic acid functional group or a sulfuric acid functional group is at least one natural polysaccharide selected from the group consisting of alginate, pectin, and carrageenan.

14. A method of adsorbing phosphoric acid comprising using the adsorbent for phosphoric acid of any one of claims 1 to 7.

FIG. 1

## INTERNATIONAL SEARCH REPORT

| International application No. |
|---|
| PCT/JP02/13248 |

| A. CLASSIFICATION OF SUBJECT MATTER |
|---|
| Int.Cl⁷  B01J20/24, A61K31/731, A61K31/732, A61K31/734, A61P3/00 |

According to International Patent Classification (IPC) or to both national classification and IPC

| B. FIELDS SEARCHED |
|---|

Minimum documentation searched (classification system followed by classification symbols)
Int.Cl⁷  B01J20/24, A61K31/731, A61K31/732, A61K31/734, A61P3/00

Documentation searched other than minimum documentation to the extent that such documents are included in the fields searched

| Jitsuyo Shinan Koho | 1926–1996 | Toroku Jitsuyo Shinan Koho | 1994–2003 |
|---|---|---|---|
| Kokai Jitsuyo Shinan Koho | 1971–2003 | Jitsuyo Shinan Toroku Koho | 1996–2003 |

Electronic data base consulted during the international search (name of data base and, where practicable, search terms used)
WPI

| C. DOCUMENTS CONSIDERED TO BE RELEVANT |
|---|

| Category* | Citation of document, with indication, where appropriate, of the relevant passages | Relevant to claim No. |
|---|---|---|
| X<br>Y | JP 6-277504 A  (Ebara Infiruko Kabushiki Kaisha),<br>04 October, 1994 (04.10.94),<br>Claims; page 2, right column, Par. No. [0008] to<br>page 3, left column, Par. No. [0017]<br>(Family: none) | 1,2,7,14<br>3-6,8-13 |
| X<br>Y | JP 7-039754 A  (Ebara Research Co., Ltd.),<br>10 February, 1995 (10.02.95),<br>Claims; page 3, left column, Par. No. [0010] to<br>right column, Par. No. [0013]<br>(Family: none) | 1,2,7,14<br>3-6,8-13 |
| Y | JP 5-155776 A  (Otsuka Pharmaceutical Factory,<br>Inc.),<br>22 June, 1993 (22.06.93),<br>Claims; page 2, right column, Par. No. [0006] to<br>page 3, left column, Par. No. [0014]<br>(Family: none) | 3-6,8-13 |

☒ Further documents are listed in the continuation of Box C.  ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 03. February, 2003 (03.02.03) | 18 February, 2003 (18.02.03) |

| Name and mailing address of the ISA/ | Authorized officer |
|---|---|
| Japanese Patent Office | |
| Facsimile No. | Telephone No. |

Form PCT/ISA/210 (second sheet) (July 1998)

## INTERNATIONAL SEARCH REPORT

| International application No. |
| --- |
| PCT/JP02/13248 |

C (Continuation). DOCUMENTS CONSIDERED TO BE RELEVANT

| Category* | Citation of document, with indication, where appropriate, of the relevant passages | Relevant to claim No. |
| --- | --- | --- |
| A | JP 6-157324 A (Tatsuaki YAMAUCHI),<br>03 June, 1994 (03.06.94),<br>Full text<br>(Family: none) | 1-14 |
| A | US 6174442 B1 (VIFOR (INTERNATIONAL) AG),<br>16 January, 2001 (16.01.01),<br>Full text<br>& JP 2000-506372 A     & EP 868125 A<br>& WO 97/22266 A1 | 1-14 |
| A | US 5514281 A (B. BRAUN MELSUNGEN AG),<br>07 May, 1996 (07.05.96),<br>Full text<br>& JP 7-075669 A      & EP 600347 A2 | 1-14 |

Form PCT/ISA/210 (continuation of second sheet) (July 1998)